# EUROPEAN PATENT APPLICATION

(11) **EP 4 137 114 A1**
(43) Date of publication of application: **22.02.2023**
(21) Application number: 21788125.9
(22) Date of filing: 30.03.2021
(51) Int. Cl.: A61J 1/20, A61M 39/10, A61M 5/178, A61J 1/06

(54) **MULTI-VIAL CONNECTOR**

(30) Priority: 17.04.2020 KR 20200046560
(71) Applicant: STS BIO, Icheon 21990 (KR)
(72) Inventor: PARK, Jung Gun, Hwaseong-si Gyeonggi-do 18489 (KR); KIM, Ho Yeon, Incheon 21030 (KR)
(74) Representative: M. Zardi & Co S.A.
(86) International application number: PCT/KR2021/003907
(87) International publication number: WO 2021/210809

(57) **Abstract**

The present invention relates to a multi-vial connector comprising: a main body, the upper side of which is coupled to a liquid medication extraction device; a plurality of sub-bodies arranged around the main body; a spike formed at the sub-body and inserted into a vial; and a liquid medication flow channel extending from the spike to the main body through the sub-body. According to the present invention, a liquid medication can be extracted from a plurality of vials at the same time in a safe and convenient manner. Therefore, the present invention can improve the safety, convenience, and rapidity for extracting a liquid medication.

## Description

### [Technical Field]

The present invention relates to a multi-vial connector connected to a plurality of vials to simultaneously extract liquid medications from the vials.

### [Background Art]

In general, intravenous injection refers to direct administration of a drug prescribed by a doctor into a vein of a patient, and the drug injected directly into a blood vessel may pass through a heart and rapidly reach necessary tissues of a body so that an effect and a response of the administered drug may appear rapidly.

The intravenous injection is mainly performed when the prescribed drug is a liquid medication that may not be injected subcutaneously or intramuscularly, when an amount of a liquid medication is too excessive even though the prescribed drug may be injected, or when a rapid effect of the drug is expected.

For such intravenous injection, a nurse has to first prepare an injectable solution in which an infusion and a liquid medication are mixed by injecting a prescribed liquid medication into an infusion container in which the infusion is stored.

In other words, the nurse may extract a prescribed amount of the liquid medication from a vial in which the liquid medication is stored by using a syringe to fill the syringe with the liquid medication, and insert an injection needle of the syringe into the infusion container to inject the liquid medication filled in the syringe into the infusion container, so that the stored infusion and the liquid medication injected through the syringe may be mixed with each other in the infusion container, and thus the injectable solution to be administered may be prepared.

In this case, the vial refers to a medication bottle or a storage container in which the liquid medication is stored in a sterile state. In general, the vial may have a structure in which an inlet on an upper end of the vial is closed with a rubber cap after the liquid medication is stored in the vial and subject to an aseptic treatment, and may have a shape that may be safely stored and transported.

In the past, the vial was mainly used in a large capacity of about 100 ml. However, after a predetermined amount of a liquid medication is extracted through a syringe, all the remaining liquid medication had to be discarded for hygiene reasons, so that a large amount of the liquid medication may be disposed of in a case of the large-capacity vial so as to excessively waste the liquid medication. Accordingly, recently, a small-capacity vial of about 20 ml have been mainly used.

However, when the small-capacity vial is used, while the waste of the liquid medication may be minimized, since an amount of a liquid medication in one vial is too small, in order to extract a prescribed amount of the liquid medication, an injection needle of a syringe has to be inserted into multiple vials one by one to extract liquid medications over multiple times. Accordingly, a process of extracting the liquid medication from the multiple vials may be cumbersome and time-consuming, and since a process of inserting or extracting the injection needle into or from the vial is repeatedly performed, a risk that the nurse may be pierced by the injection needle or the liquid medication may be exposed may be increased.

### [Disclosure]

### [Technical Problem]

To solve the problems of the related art as described above, an object of the present invention is to provide a multi-vial connector in which a liquid medication may be extracted from a vial without a concern that piercing by an injection needle may occur or the liquid medication may be contaminated, and in particular, the multi-vial connector may be connected to a plurality of vials to simultaneously extract liquid medications from the vials so that rapidity and convenience as well as safety may be improved.

### [Technical Solution]

To achieve the objects described above, according to the present invention, there is provided a multi-vial connector including: a main body having an upper side coupled to a liquid medication extraction device; a plurality of sub-bodies arranged around the main body; a spike formed on the sub-body and inserted into a vial; and a liquid medication flow channel extending from the spike to the main body via the sub-body.

In this case, three sub-bodies may be provided, and arranged at an interval of 120 degrees radially around the main body so as to be connected to the main body.

In addition, the liquid medication flow channel may include: a liquid medication inlet formed in the spike; a liquid medication outlet formed on an inner upper side of the main body; a sub-liquid medication flow channel extending along an inside of the sub-body while communicating with the liquid medication inlet; and a main liquid medication flow channel extending upward from an inside of the main body while being connected to the sub-liquid medication flow channel to communicate with the liquid medication outlet.

In addition, the multi-vial connector may further include a ventilation flow channel extending from the spike to the main body via the sub-body independently of the liquid medication flow channel.

In addition, the ventilation flow channel may include: a first ventilation hole formed in the spike; a second ventilation hole formed on an inner lower side of the main body; a sub-ventilation flow channel extending along an inside of the sub-body while communicating with the first ventilation hole; and a main ventilation flow channel extending downward from an inside of the main body while being connected to the sub-ventilation flow channel to communicate with the second ventilation hole.

In addition, the multi-vial connector may further include a head cap provided in the sub-body and detachably coupled to a head part of the vial.

In addition, the head cap may include: a cap body having a cylindrical shape and having an open lower portion; a mounting end formed on an upper end of the cap body so as to be coupled to the sub-body; a plurality of incision slits formed by cutting a side surface of the cap body at regular intervals; an inclined surface formed on an inner lower side of the cap body; and a latching sill formed on an upper end of the inclined surface.

### [Advantageous Effects]

According to the multi-vial connector of the present invention,
the multi-vial connector may be connected to the vial through the insertion of the spike while being coupled to a syringe, so that the liquid medication can be safely extracted from the vial without a concern that piercing by an injection needle may occur or the liquid medication may be contaminated.

In particular, the multi-vial connector may be connected to a plurality of vials to simultaneously extract liquid medications from the vials, so that an operation can be performed more rapidly and conveniently.

In addition, a pressure inside the vial may be constantly maintained by the ventilation flow channel during the extraction of the liquid medication, so that the liquid medication can be extracted more easily without effort.

### [Description of Drawings]

FIG. 1 is a view three-dimensionally illustrating an overall configuration of the present invention.
FIG. 2 is a view three-dimensionally illustrating the overall configuration of the present invention from a bottom side.
FIG. 3 is a view illustrating a configuration in which a head cap is removed from FIG. 2.
FIG. 4 is a view illustrating a head cap according to one embodiment of the present invention.
FIG. 5 is a sectional view illustrating a liquid medication flow channel and a ventilation flow channel according to the present invention.
FIG. 6 is a view illustrating a use state of the present invention.
FIG. 7 is a view illustrating an operation relation of the present invention.

### [Mode for Invention]

Hereinafter, an exemplary embodiment of the present invention will be described in detail with reference to the accompanying drawings.

In the present disclosure, terms indicating directions such as front, rear, left, right, upper, and lower have been used only to describe directions shown and observed in the drawings, so that the terms may vary when the directions shown and observed in the drawing are changed.

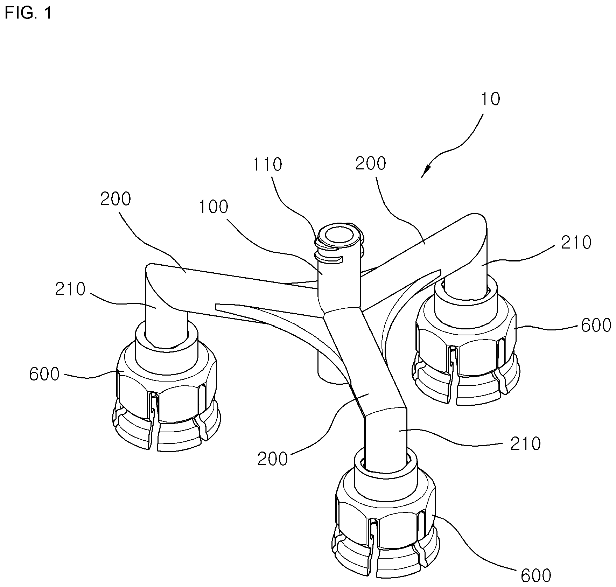

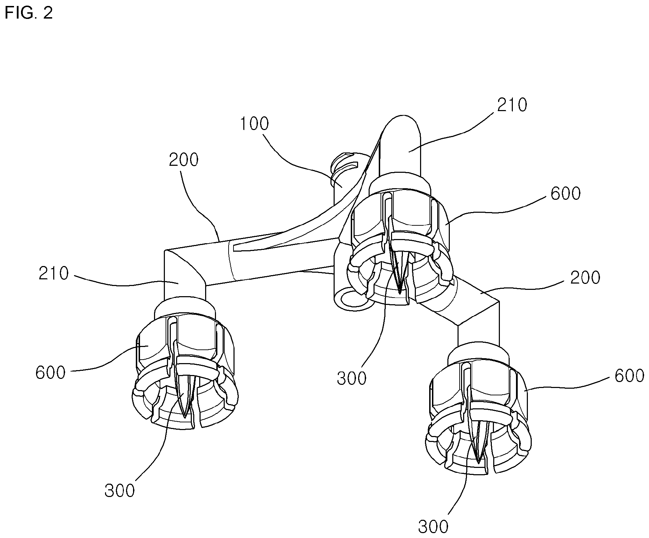

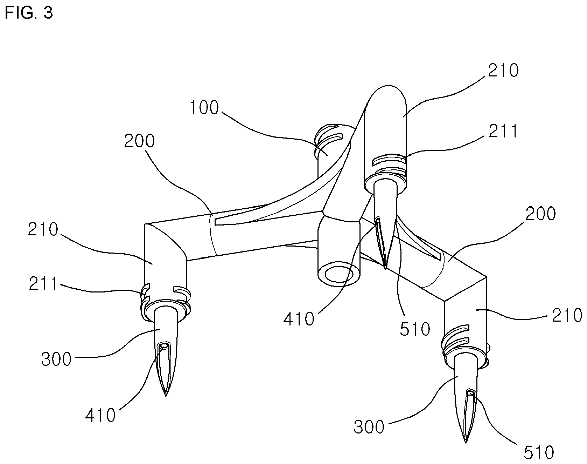

FIGS. 1 to 7 illustrate a configuration and an operation of a multi-vial connector according to one embodiment of the present invention.

Referring to the drawings, according to the present invention, a multi-vial connector 10 (hereinafter abbreviated as "connector") may include a main body 100, a sub-body 200, a spike 300, a liquid medication flow channel 400, a ventilation flow channel 500, and a head cap 600.

The main body 100 refers to a part coupled to a liquid medication extraction device 20, and the main body 100 may be formed on an upper side thereof with a coupling screw 110 for detachable coupling with the liquid medication extraction device 20.

In this case, the liquid medication extraction device 20 to which the main body 100 is coupled refers to a device capable of extracting a liquid medication from a vial 30 by a pressure difference, is not particularly limited, and may be, for example, a syringe.

The main body 100 may have a cylindrical shape extending vertically by a predetermined length, but is not limited thereto.

The sub-body 200 refers to a part connected to the main body 100.

The sub-body 200 may extend by a predetermined length in a direction that is different from a longitudinal direction of the main body 100. As illustrated in the drawings, the sub-body 200 may extend in a horizontal direction that is perpendicular to the longitudinal direction (i.e., a vertical direction) of the main body 100, but is not limited thereto, and may extend so as to be inclined downward or upward at a predetermined angle.

Meanwhile, the sub-body 200 may have a leg part 210 at a point extended by a predetermined length, and the leg part 210 may be bent in the same direction as the longitudinal direction of the main body 100, that is, downward from the sub-body 200, which extends by the predetermined length from the main body 100, so as to extend by a predetermined length.

The leg part 210 may include a coupling screw 211 for detachable coupling with a head cap 600 that will be described below.

The sub-body 200 may have a cylindrical shape similarly to the main body 100, but is not limited thereto.

A plurality of sub-bodies 200, which are at least two sub-bodies 200, may be provided, and the sub-bodies 200 may be arranged at an interval of a predetermined degree radially around the main body 100 so as to be connected to the main body 100.

For example, as illustrated in the drawings, three sub-bodies 200 may be provided. In this case, the three sub-bodies 200 may be arranged at an interval of 120° radially around the main body 100 so as to be connected to the main body 100.

Meanwhile, two or four sub-bodies 200 may be provided. In this case, the two sub-bodies 200 may be arranged at an interval of 180°, and the four sub-bodies 200 may be arranged at an interval of 90°.

The spike 300 refers to a part inserted into and connected to the vial 30.

Therefore, the spike 300 may have a predetermined length in which a tip has a pointed shape as an outer diameter is gradually reduced, so that the spike 300 may be inserted into the vial 30 by penetrating through a rubber cap provided in a head part of the vial 30.

The spike 300 may be formed on each of the sub-bodies 200.

The spike 300 may extend in the same direction as a longitudinal direction of the sub-body 200.

However, the spike 300 may preferably extend in the same direction as the longitudinal direction of the main body 100, that is, downward so that the liquid medication extraction device 20 and the vial 30 coupled to the connector 10 may be connected to each other in the same direction. Therefore, the spike 300 may extend downward from a lower end of the leg part 210 of the sub-body 200.

The liquid medication flow channel 400 may perform a function of a flow channel through which the liquid medication extracted from the vial 30 may move to the liquid medication extraction device 20.

The liquid medication flow channel 400 may be formed as a path extending from the spike 300 to an inside of the main body 100 via an inside of the sub-body 200.

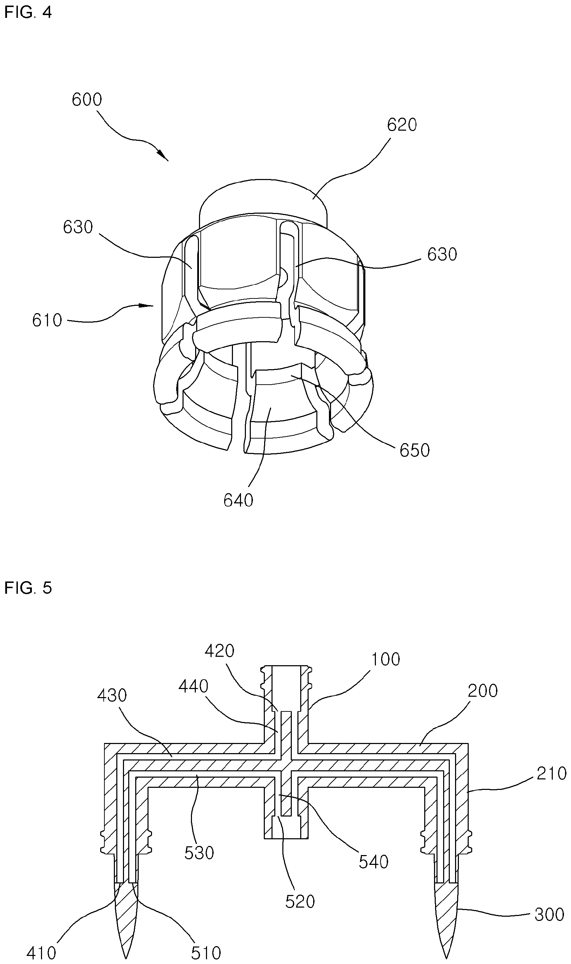

In detail, as illustrated in FIG. 5, the liquid medication flow channel 400 may include: a liquid medication inlet 410 formed in the spike 300; a liquid medication outlet 420 formed on an inner upper side of the main body 100; a sub-liquid medication flow channel 430 extending along an inside of the sub-body 200 while communicating with the liquid medication inlet 410; and a main liquid medication flow channel 440 extending upward from an inside of the main body 100 while being connected to the sub-liquid medication flow channel 430 to communicate with the liquid medication outlet 420.

In this case, a plurality of sub-liquid medication flow channels 430 may be provided to correspond to a number of the sub-bodies 200, so that a plurality of main liquid medication flow channels 440 may be provided to correspond to the sub-liquid medication flow channels 430 so as to be connected in one-to-one correspondence with the sub-liquid medication flow channels 430. However, a single main liquid medication flow channel 440 may be provided so as to be simultaneously connected to the sub-liquid medication flow channels 430.

The ventilation flow channel 500 may perform a function of a flow channel for circulating external air into the vial 30.

When the spike 300 is inserted into the vial 300, an inside of the vial 300 may be ventilated with an outside through the ventilation flow channel 500, so that a pressure inside the vial 300 may be constantly maintained at an atmospheric pressure regardless of a change in an amount of the liquid medication, and thus the liquid medication may be easily extracted.

The ventilation flow channel 500 may be formed independently of the liquid medication flow channel 400, and may be formed as a path extending from the spike 300 to the inside of the main body 100 via the inside of the sub-body 200.

In detail, as illustrated in FIG. 5, the ventilation flow channel 500 may include: a first ventilation hole 510 formed in the spike 300; a second ventilation hole 520 formed on an inner lower side of the main body 100; a sub-ventilation flow channel 530 extending along an inside of the sub-body 200 while communicating with the first ventilation hole 510; and a main ventilation flow channel 540 extending downward from an inside of the main body 100 while being connected to the sub-ventilation flow channel 530 to communicate with the second ventilation hole 520.

In this case, a plurality of sub-ventilation flow channels 530 may be provided to correspond to the number of the sub-bodies 200, so that a plurality of main ventilation flow channels 540 may be provided so as to be connected in one-to-one correspondence with the sub-ventilation flow channels 530, or a single main ventilation flow channel 540 may be provided so as to be simultaneously connected to the sub-ventilation flow channels 530.

In addition, although not shown in the drawings, a filter member capable of filtering contaminants in the air may be further installed in the second ventilation hole 520 communicating with the outside.

The head cap 600 refers to a part coupled to the head part of the vial 30 to fix the connector 10 according to the present invention to the vial 30 when the spike 300 is inserted into the vial 30 to extract the liquid medication.

The head cap 600 may be provided on each of the sub-bodies 200, and particularly, the head cap 600 may be mounted on the leg part 210 of the sub-body 200.

As illustrated in FIG. 4, the head cap 600 may include: a cap body 610 having a cylindrical shape and having an open lower portion; and a mounting end 620 protruding from an upper end of the cap body 610 so as to be fastened to the coupling screw 211 of the leg part 210.

The cap body 610 may be formed on a side surface thereof with a plurality of incision slits 630 at regular intervals, and formed on an inner lower side thereof with an inclined surface 640, and a latching sill 650 may be formed on an upper end of the inclined surface 640.

When the spike 300 is inserted into the rubber cap of the vial 30 to extract the liquid medication, the head cap 600 may be fitted around the head part of the vial 30 as the cap body 610 is elastically spread by the inclined surface 640 and the incision slits 630, and may be elastically restored to allow the latching sill 650 to be latched and supported at a lower end of the head part, so that the connector 10 according to the present invention may be fixed to the vial 30.

Since the vial 30 is fixed to the connector 10 by the head cap 600 as described above, the vial 30 may be prevented from being arbitrarily separated during the extraction of the liquid medication, so that the liquid medication may be safely extracted.

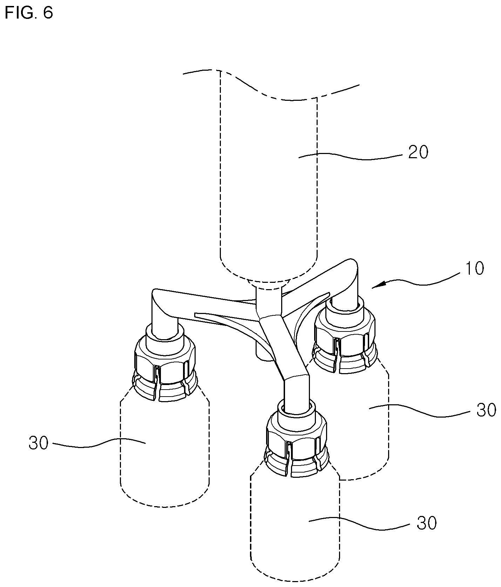

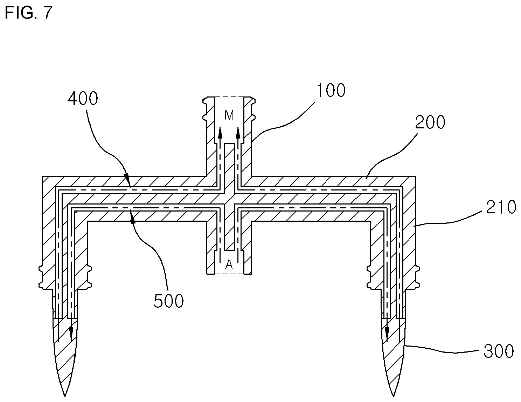

The above-described operation of the present invention will be briefly described with reference to FIGS. 6 and 7, while one example in which the connector 10 includes three sub-bodies 200 will be described for convenience of description.

The connector 10 according to the present invention may be used when a predetermined amount of a liquid medication is extracted from the vial 30 by using the liquid medication extraction device 20 according to a prescription of a doctor, and particularly, the connector 10 according to the present invention may be used when a relatively large amount of the liquid medication is extracted from a plurality of small-capacity vials 30.

While the syringe, which is the liquid medication extraction device 20, and three vials 30 in which a predetermined amount of the liquid medication is stored are prepared, first, the syringe 20 may be coupled to an upper end of a main body 10.

In this case, the syringe 20 may be firmly coupled by using the coupling screw 110 formed on an upper side of the main body 10.

Thereafter, while the spikes 300 formed on the three sub-bodies 200 are sequentially inserted into the three vials 30, the head cap 600 may be fitted around the head part of the vial 30.

Then, while the spike 300 penetrates through the rubber cap to enter the inside of the vial 30, the head cap 600 may be elastically spread and restored so as to be coupled to the head part of the vial 30, so that the three sub-bodies 200 may be connected and fixed to the three vials 30, respectively.

As described above, when the main body 100 is coupled to the syringe 20, and the three sub-bodies 200 are coupled to the three vials 30, respectively, preparation for extracting the liquid medication may be completed, and FIG. 6 illustrates a state in which the preparation for extracting the liquid medication is completed as described above.

Thereafter, when a push rod of the syringe 20 is pulled so as to be moved rearward, as a negative pressure is generated inside the syringe 20, the liquid medication M may be simultaneously extracted from the three vials 30, and as illustrated in FIG. 7, the liquid medication M extracted from the three vials 30 as described above may move along the liquid medication flow channel 400 so as to be filled in the syringe 20.

In this case, since the air A is circulated into the vial 30 by the ventilation flow channel 500, the pressure inside the vial 30 may be constantly maintained at the atmospheric pressure regardless of the change in the amount of the liquid medication, so that the liquid medication M may be easily extracted.

In other words, since the inside of the vial 30 is sealed, the negative pressure may be generated to correspond to an amount of the liquid medication extracted and escaped from the vial 30, so that it may be difficult to continuously extract the liquid medication. However, according to the present invention, the pressure inside the vial 30 may be constantly maintained by the ventilation flow channel 500, so that the liquid medication may be easily extracted.

As described above, according to the connector 10 of the present invention, the spike 300 may be inserted into the vial 30 to extract the liquid medication without using an injection needle, and the pressure inside the vial 30 may be constantly maintained during the extraction of the liquid medication, so that the liquid medication may be safely and easily extracted.

In particular, liquid medications may be simultaneously extracted from a plurality of vials 30 instead of sequentially extracting the liquid medications from the vials 30, so that cumbersomeness and inconvenience of an extraction operation may be minimized, and an operation time may be greatly shortened.

Although the exemplary embodiment of the present invention has been described in detail above, the technical scope of the present invention is not limited to contents set forth in the embodiment and the drawings described above, an equivalent configuration modified or changed by a person having ordinary skill in the art can be made without departing from the scope of the technical idea of the present invention.

## Claims

1. A multi-vial connector comprising:
a main body (100) having an upper side coupled to a liquid medication extraction device (20);
a plurality of sub-bodies (200) arranged around the main body (100);
a spike (300) formed on the sub-body (200) and inserted into a vial (30); and
a liquid medication flow channel (400) extending from the spike (300) to the main body (100) via the sub-body (200).

2. The multi-vial connector of claim 1, wherein three sub-bodies (200) are provided, and arranged at an interval of 120 degrees radially around the main body (100) so as to be connected to the main body (100).

3. The multi-vial connector of claim 1, wherein the liquid medication flow channel (400) includes:
a liquid medication inlet (410) formed in the spike (300);
a liquid medication outlet (420) formed on an inner upper side of the main body (100);
a sub-liquid medication flow channel (430) extending along an inside of the sub-body (200) while communicating with the liquid medication inlet (410); and
a main liquid medication flow channel (440) extending upward from an inside of the main body (100) while being connected to the sub-liquid medication flow channel (430) to communicate with the liquid medication outlet (420).

4. The multi-vial connector of claim 1, further comprising a ventilation flow channel (500) extending from the spike (300) to the main body (100) via the sub-body (200) independently of the liquid medication flow channel (400).

5. The multi-vial connector of claim 4, wherein the ventilation flow channel (500) includes:
a first ventilation hole (510) formed in the spike (300);
a second ventilation hole (520) formed on an inner lower side of the main body (100);
a sub-ventilation flow channel (530) extending along an inside of the sub-body (200) while communicating with the first ventilation hole (510); and
a main ventilation flow channel (540) extending downward from an inside of the main body (100) while being connected to the sub-ventilation flow channel (530) to communicate with the second ventilation hole (520).

6. The multi-vial connector of claim 1, further comprising a head cap (600) provided in the sub-body (200) and detachably coupled to a head part of the vial (30).

7. The multi-vial connector of claim 6, wherein the head cap (600) includes:
a cap body (610) having a cylindrical shape and having an open lower portion;
a mounting end (620) formed on an upper end of the cap body (610) so as to be coupled to the sub-body (200);
a plurality of incision slits (630) formed by cutting a side surface of the cap body (610) at regular intervals;
an inclined surface (640) formed on an inner lower side of the cap body (610); and
a latching sill (650) formed on an upper end of the inclined surface (640).
